# EUROPEAN PATENT SPECIFICATION

(11) **EP 3 349 859 B1**
(45) Date of publication and mention of the grant of the patent: **23.09.2020**
(21) Application number: 15784529.8
(22) Date of filing: 08.10.2015
(51) Int. Cl.: A61Q 11/00, A61K 8/27, A61K 8/25, A61K 8/19, A61K 8/26, A61K 8/24, A61K 8/21

(54) **ORAL CARE COMPOSITIONS**
MUNDPFLEGEMITTEL
PRODUITS D'HYGIÈNE BUCCALE

(43) Date of publication of application: 25.07.2018
(73) Proprietor: Colgate-Palmolive Company, New York, NY 10022 (US)
(72) Inventor: REGE, Aarti, East Windsor, New Jersey 08520 (US); SURIANO, David, Edison, New Jersey 08817 (US); SULLIVAN, Richard, Atlantic Highlands, New Jersey 07716 (US)
(74) Representative: Wibbelmann, Jobst
(86) International application number: PCT/US2015/054791
(87) International publication number: WO 2017/062020

(56) References cited:
- GB-A- 845 611

## Description

### BACKGROUND

Dental erosion involves demineralization and damage to the tooth structure due to acid attack from nonbacterial sources. Erosion is found initially in the enamel and, if unchecked, may proceed to the underlying dentin. Dental erosion may be caused or exacerbated by acidic foods and drinks, exposure to chlorinated swimming pool water, and regurgitation of gastric acids.

The tooth enamel is a negatively charged surface, which naturally tends to attract positively charged ions such as hydrogen and calcium ions, while resisting negatively charged ions such as fluoride ions. Depending upon relative pH of surrounding saliva, the tooth enamel will lose or gain positively charged ions such as calcium ions. Generally saliva has a pH between 7.2 and 7.4. When the pH is lowered the fluid medium surrounding the tooth becomes undersaturated with respect to tooth mineral phase and the tooth dissolves, releasing calcium and phosphate ions. This damages the enamel and creates a porous, sponge-like roughened surface. If saliva remains acidic over an extended period, then remineralization may not occur, and the tooth will continue to lose minerals, causing the tooth to weaken and ultimately to lose structure.

Heavy metal ions, such as zinc and stannous, are resistant to acid attack. These ions have been shown to form insoluble compounds that deposit on the surface of teeth to form a film that can protect tooth enamel from erosion, as taught in U.S. Patent Applicant No. 2003/0165442, the disclosure of which is hereby incorporated by reference in its entirety. Also, stannous has been shown to have anti-microbial properties in plaque and caries studies and is known for providing antigingivitis benefits.

Soluble stannous salts, such as stannous fluoride, stannous chloride, stannous acetate, sodium stannous fluoride, potassium stannous fluoride, stannous hexafluorozirconate, stannous sulphate, stannous tartrate, stannous gluconate and others, have been used in dentifrice compositions, but have disadvantages. Stannous ions in solution impart an unpleasant, astringent mouthfeel, so formulations that provide effective levels of stannous, and also have acceptable therapeutic properties, can been difficult to achieve. Moreover, free stannous ions are unstable in aqueous solutions. The instability of stannous fluoride in water is primarily due to the reactivity of the stannous ion (Sn²). Tin readily hydrolyses above a pH of 4, resulting in precipitation from solution, with a consequent loss of therapeutic properties.

Thus, there is a desire for providing stable stannous formulations that can provide one or more of the therapeutic benefits of stannous. There is also a desire for providing improved products for treating or controlling erosion of tooth enamel, increasing antimicrobial effectiveness, reducing plaque or treating or controlling gingivitis. See e.g. the disclosure provided in GB845611.

### BRIEF SUMMARY

The present disclosure is directed to an oral care composition. The composition comprises at least one metal phosphate chosen from stannous phosphate, magnesium phosphate or aluminum phosphate; and at least one fluoride ion source. The at least one phosphate is added to the oral care composition as a preformed salt.

Further areas of applicability of the present disclosure will become apparent from the detailed description provided hereinafter. It should be understood that the detailed description and specific examples, while indicating the preferred embodiment of the disclosure, are intended for purposes of illustration only and are not intended to limit the scope of the disclosure. In the context of this disclosure, the invention is strictly limited by the scope of the appended claims.

### DETAILED DESCRIPTION

The following description of the preferred embodiment(s) is merely exemplary in nature and is in no way intended to limit the disclosure, its application, or uses.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

An embodiment of the present disclosure is directed to an oral care composition for intermittent use, e.g., daily use, in the form of a dentifrice. The oral care composition comprises: at least one metal phosphate chosen from stannous phosphate, magnesium phosphate or aluminum phosphate, wherein the at least one phosphate is added to the oral care composition as a preformed salt; and at least one fluoride ion source. As used herein, the term "preformed salt" means that the phosphate is not formed in situ in the oral care composition, e.g., through the reaction of phosphoric acid and a stannous salt.

In an embodiment, the metal phosphate is an inorganic phosphate salt of stannous, magnesium or aluminum. "Phosphate" as used herein encompasses orally acceptable mono- and polyphosphates, for example, P₁₋₆ phosphates, for example monomeric phosphates such as monobasic, dibasic or tribasic phosphate; and dimeric phosphates such as pyrophosphates; and multimeric phosphates. In an embodiment, the metal phosphate is an anhydrous or hydrated stannous phosphate salt, such as, for example, tribasic stannous phosphate. In an embodiment, the phosphate is not a pyrophosphate, metaphosphate or other polyphosphate. Preformed stannous salts other that stannous phosphate can be employed in addition to the stannous phosphate. In an alternative embodiment, the only preformed stannous compound employed in the composition is stannous phosphate. In an embodiment, the stannous phosphate compound does not include an organic ligand.

Any amount of metal phosphate that is effective for protecting against enamel erosion and/or providing any of the other benefits described herein can be employed. Examples of suitable amounts of metal phosphate can range from 0.1 to 10 % by weight, such as from 0.2 to 5% by weight, such as from 0.5 to 4% by weight, relative to the weight of the dentifrice composition.

The metal phosphate, when placed in formulation, e.g., at acidic or basic pH, can dissolve sufficiently upon use to provide an effective concentration of metal ions to the enamel, thereby protecting against erosion, reducing bacterial colonization and biofilm development, and providing enhanced shine to the teeth. In some embodiments, the formulation comprises an amino acid, such as a basic amino acid, e.g., arginine or lysine, which can confer a basic pH to the formulation.

The compositions of the present disclosure also include at least one fluoride ion source, such as a fluoride salt. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride and sodium fluoride, as well as mixtures thereof. These fluoride ion sources in combination with stannous phosphate have been found by the inventors of the present disclosure to provide enhanced anti-microbial and/or anti-erosion properties. The addition of the at least one fluoride ion may potentially provide one or more other advantages, such as reducing harmful plaque and bacteria.

In certain embodiments, the oral care composition of the disclosure may contain a source of fluoride ions or fluorine-providing ingredient in amounts sufficient to supply from 25 ppm to 25,000 ppm (mass fraction) of fluoride ions, generally at least 500 ppm, e.g., from 500 to 2000 ppm, e.g., from 1000 to 1600 ppm, e.g., about 1450 ppm. The appropriate level of fluoride will depend on the particular application. Toothpaste for general consumer use would typically have from 1000 to 1 500 ppm, with pediatric toothpaste having somewhat less. A dentifrice or coating for professional application could have as much as 5,000 or even 25,000 ppm fluoride. Fluoride ion sources may be added to the compositions of the disclosure at a level of from 0.01 wt. % to 10 wt. % in one embodiment or from 0.03 wt. % to 5 wt. %, and in another embodiment from 0.1 wt. % to 1 wt. % by total weight of the composition. Weights of fluoride salts to provide the appropriate level of fluoride ion will obviously vary based on the weight of the counter ion in the salt.

The compositions may optionally comprise additional ingredients suitable for use in oral care compositions. Examples of such ingredients include active agents, such as an additional fluoride source and/or an additional phosphate source. The compositions may be formulated in a dentifrice base, e.g., comprising abrasives, e.g., silica abrasives, surfactants, foaming agents, vitamins, polymers, enzymes, humectants, thickeners, additional antimicrobial agents, preservatives, flavorings, colorings, and/or combinations thereof. Examples of suitable dentifrice bases are known in the art. Alternatively, compositions may be formulated as a gel (e.g., for use in tray), chewing gum, lozenge or mint. Examples of suitable additional ingredients that can be employed in the compositions of the present disclosure are discussed in more detail below.

*Active Agents:* The compositions of the disclosure may comprise various other agents which are active to protect and enhance the strength and integrity of the enamel and tooth structure and/or to reduce bacteria and associated tooth decay and/or gum disease. Effective concentration of the active ingredients used herein will depend on the particular agent and the delivery system used. The concentration will also depend on the exact salt or polymer selected. For example, where the active agent is provided in salt form, the counter ion will affect the weight of the salt, so that if the counter ion is heavier, more salt by weight will be required to provide the same concentration of active ion in the final product. Arginine, where present, may be present at levels from, e.g., from 0.1 to 20 wt. % (expressed as weight of free base), e.g., from 1 to 10 wt. % for a consumer toothpaste or from 7 to 20 wt. % for a professional or prescription treatment product. In an embodiment, a triclosan toothpaste may contain from 0.1 to 1 wt. %, such as about 0.3 wt. % triclosan.

*Amino acids*: In some embodiments, the compositions of the disclosure comprise an amino acid, In particular embodiments, the amino acid may be a basic amino acid. By "basic amino acid" is meant the naturally occurring basic amino acids, such as arginine, lysine, and histidine, as well as any basic amino acid having a carboxyl group and an amino group in the molecule, which is water-soluble and provides an aqueous solution with a pH of from 7 or greater. Accordingly, basic amino acids include, but are not limited to, arginine, lysine, citrulline, ornithine, creatine, histidine, diaminobutanoic acid, diaminoproprionic acid, salts thereof or combinations thereof. In a particular embodiment, the basic amino acids are selected from arginine, citrulline, and ornithine. In certain embodiments, the basic amino acid is arginine, for example, 1-arginine, or a salt thereof. In other embodiments, the amino acid is quaternized, e.g., the amino group is additionally substituted to form a quaternary ammonium moiety, which may form an inner salt with the carboxyl group, for example, betaine (*N,N,N*-trimethylglycine).

In various embodiments, the amino acid is present in an amount of from 0.5 wt. % to 20 wt. % of the total composition weight, from 0.5 wt. % to 10 wt. % of the total composition weight, for example 1.5 wt. %, 3.75 wt. %, 5 wt. %, or 7.5 wt. % of the total composition weight in the case of a dentifrice.

*Abrasives*: The compositions of the disclosure can include abrasives, which may include any suitable silica abrasive, such as standard cleaning silicas, high cleaning silicas and/or other abrasive silicas. Other suitable additional abrasives include, for example, a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof.

Silica abrasive polishing materials useful herein, as well as the other abrasives, generally have an average particle size ranging between from 0.1 and 30 microns, such as between 5 and 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason. In certain embodiments, abrasive materials useful in the practice of the oral care compositions in accordance with the disclosure include silica gels and precipitated amorphous silica having an oil absorption value of less than 100 cc/100 g silica and in the range of from 45 cc/100 g to 70 cc/100 g silica. Oil absorption values are measured using the ASTA Rub-Out Method D281. In certain embodiments, the silicas are colloidal particles having an average particle size of from 3 microns to 12 microns, and from 5 to 10 microns. Low oil absorption silica abrasives particularly useful in the practice of the disclosure are marketed under the trade designation Sylodent XWA®) by Davison Chemical Division of W.R. Grace & Co., Baltimore, Md. 21203. Sylodent 650 XWA®, a silica hydrogel composed of particles of colloidal silica having a water content of 29% by weight averaging from 7 to 10 microns in diameter, and an oil absorption of less than 70 cc/100 g of silica is an example of a low oil absorption silica abrasive useful in the practice of the present disclosure.

Any suitable amount of silica abrasive can be employed. Examples of suitable amounts include 5 wt. % or more dry weight of silica particles, such as from 5 wt. % to 40 wt. %, or from 15 wt. % to 30 wt. % or from 15 wt. % to 25 wt. %, based on the total weight of the composition.

*Foaming agents*: The oral care compositions of the disclosure also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care compositions of the present disclosure. Polyoxyethylene is also commonly known as polyethylene glycol ("PEG") or polyethylene oxide. The polyoxyethylenes suitable for compositions of the present disclosure will have a molecular weight of from 200,000 to 7,000,000. In one embodiment, the molecular weight will be from 600,000 to 2,000,000 and in another embodiment from 800,000 to 1,000,000. Polyox® is the trade name for the high molecular weight polyoxyethylene produced by Union Carbide. The foaming agent, (e.g., polyoxyethylene) may be present in an amount of from 0.1% to 50%, in one embodiment from 0.5% to 20% and in another embodiment from 1% to 10%, or from 2% to 5% by weight of the oral care compositions of the present disclosure.

*Surfactants:* The compositions of the present disclosure may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₘOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na),
iv. higher alkyl aryl sulfonates such as sodium dodecyl benzene sulfonate (sodium lauryl benzene sulfonate),
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfacetamide) and sodium lauryl sarcosinate.

By "higher alkyl" is meant, e.g., C₆₋₃₀alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. In one embodiment, the anionic surfactant is present in a toothpaste at from 0.3% to 4.5% by weight, e.g., about 1.5%. The compositions of the disclosure may optionally contain mixtures of surfactants, e.g., comprising anionic surfactants and other surfactants that may be anionic, cationic, zwitterionic or nonionic. Generally, suitable surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4.051.234, to Gieske et al. In certain embodiments, the anionic surfactants useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate, sodium lauroyl sarcosinate and sodium coconut monoglyceride sulfonates are examples of anionic surfactants of this type. In a particular embodiment, the compositions of the disclosure comprise sodium lauryl sulfate.

The surfactant or mixtures of compatible surfactants that are included in addition to the anionic surfactants can be present in the compositions of the present disclosure in an amount of from 0.1% to 5.0%, in another embodiment from 0.3% to 3.0% and in another embodiment from 0.5% to 2.0% by weight of the total composition. These ranges do not include the anionic surfactant amounts.

In an embodiment, the compositions of the present disclosure include a zwitterionic surfactant, for example a betaine surfactant, for example cocamidopropylbetaine, e.g. in an amount of from 0.1% to 4.5% by weight, e.g. 0.5-2% cocamidopropylbetaine.

*Tartar control agents:* In various embodiments of the present disclosure, the compositions comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, and diphosphonates. The disclosure thus may comprise phosphate salts in addition to the stannous phosphate. In particular embodiments, these salts are alkali phosphate salts, e.g., salts of alkali metal hydroxides or alkaline earth hydroxides, for example, sodium, potassium or calcium salts (e.g., sodium hexametaphosphate). "Phosphate" as used here is defined as above. In particular examples, the phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of from 3 to 4 wt. % of the sodium phosphate dibasic and from 0.2 to 1 wt. % of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at from 0.5 to 5 wt. %, such as from 1 to 2 wt. % and STPP at from 7% to 10%, based on the weight of the composition. Such phosphates are provided in an amount effective to reduce erosion of the enamel, to aid in cleaning the teeth, and/or to reduce tartar buildup on the teeth, for example in an amount of from 0.2 to 20 wt. %, e.g., from 1 to 15 wt. %, by weight of the composition.

*Flavoring Agents:* The oral care compositions of the disclosure may also include a flavoring agent. Flavoring agents which are used in the practice of the present disclosure include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of from 0.1 to 5% by weight e.g., from 0.5 to 1.5% by weight.

*Polymers:* The oral care compositions of the disclosure may also include additional polymers to adjust the viscosity of the formulation or enhance the solubility of other ingredients. Such additional polymers include polyethylene glycols, polysaccharides (e.g., cellulose derivatives, for example carboxymethyl cellulose, macrocrystalline cellulose or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of from 0.5% to 5.0% by weight of the total composition are used.

The compositions of the disclosure may include an anionic polymer, for example in an amount of from 0.05 to 5%. Examples of such agents generally known for use in dentifrice are disclosed in U.S. Pat. Nos. 5,188,821 and 5,192,531 and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W,) of from 30,000 to 1,000,000, such as from 300,000 to 800,000. These copolymers are available for example as Gantrez, e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from 0.05 to 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of from 1,000 to 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 issued to Sikes et al.

*Water:* The oral compositions may comprise significant levels of water, such as more than 10% by weight, such as 15% to 70% by weight or 20% to 50% by weight, based on the total weight of the composition. Water employed in the preparation of commercial oral compositions can be deionized (sometimes referred to as demineralized water) and free of organic impurities. The amount of water in the compositions includes the free water which is added plus that amount which is introduced with other materials.

*Humectants:* Within certain embodiments of the oral compositions, it is also desirable to incorporate a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In one embodiment of the disclosure, the principal humectant is one of glycerin, sorbitol or a combination thereof. The humectant may be present at levels of greater than 25 wt. %, such as 25 to 55 wt. %, or 30 wt. % to 50 wt. %, based on the total weight of the composition.

*Other optional ingredients:* In addition to the above-described components, the embodiments of this disclosure can contain a variety of optional dentifrice ingredients some of which are described below. Optional ingredients include, for example, but are not limited to, adhesives, sudsing agents, flavoring agents, sweetening agents such as sodium saccharin, additional antiplaque agents, abrasives, aesthetics such as TiO₂ coated mica or other coloring agents. These and other optional components are further described in U.S. Pat. No. 5,004,597, to Majeti; U.S. Pat. No. 3,959,458 to Agricola et al. and U.S. Pat. No. 3,937,807, to Haefele.

In an embodiment, the compositions of the present disclosure are essentially free of, or do not contain any of, one or more of the following ingredients: acyl sarcosinate or salts thereof, such as sodium lauroyl sarcosinate, sodium cocoyl sarcosinate, sodium myristoyl sarcosinate, TEA lauroyl sarcosinate, ammonium cocoyl sarcosinate, ammonium lauroyl sarcosinate; an antioxidant, such as propyl gallate, butylated hydroxyanisole (BHA), butylated hydroxytoluene (BHT), ethyl vanillin, rosemary oil, lecithin, vitamin E, rutin, morin, fisetin and other bioflavonoids; a phytic acid compound, which is defined herein to include phytic acid and salts thereof, such as alkali metal or alkaline earth metal salts and phytin, as well as myoinositolpentaphosphoric acid and salts thereof; ethylenediaminetetracetates; fused silica; a crosslinked polyvinyl pyrrolidone thickener; a potassium salt desensitizing agent chosen from potassium nitrate, potassium citrate, potassium chloride, potassium bicarbonate and potassium oxalate; a copper containing compound, such as a copper salt or other copper-ion releasing copper compound selected from the group consisting of copper sulphate, copper halides and pseudohalides, copper nitrate, copper salts of carboxylic acids in the homologous series formic acid to decanoic acid, copper salts of polybasic acids in the series oxalic acid to suberic acid, copper salts of bydroxycarboxylic acids selected from glycolic acid, lactic acid, tartaric acid, malic acid and citric acid, copper precipitated on a solid or colloidal support, such as supports selected from silica, clay, biopolymer and synthetic polymer, and copper hydrotalcite, or polyorganosilsesquioxane, which can be in the form of particles, such as polymethylsilsesquioxane. The term "essentially free" is defined herein to mean less than 0.01% by weight, based on the total weight of the composition.

The present application further discloses methods of using the compositions described herein to increase stannous levels in the enamel and to treat, reduce or control the incidence of enamel erosion, comprising applying any of the compositions as described herein to the teeth, e.g., by brushing, In various embodiments, the disclosure provides a method to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x) reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; (xv) reduce tartar build-up, and/or (xvi) promote systemic health, including cardiovascular health, e.g., by reducing potential for systemic infection via the oral tissues, comprising applying any of the compositions as described above to the oral cavity of a person in need thereof, e.g., by brushing the teeth one or more times per day with any of the compositions of the present disclosure. The disclosure further provides compositions for use in any of these methods.

### EXAMPLES

### Example 1 - Dentifrice Formulation

Test dentifrice comprising 1% stannous phosphate in combination with 0.243% sodium fluoride was prepared in accordance with the following formulation of Example 1, as shown in Table 1. The formulations of Example 2 and Comparative Example A of Table 1 were also prepared. Ingredient amounts listed are by weight of composition.

**Table 1**

| **Ingredient** | **Example 1 Wt. %** | **Comparative Example A Wt. %** | **Example 2 Wt. %** |
|---|---|---|---|
| PEG600 | 2.0 | 2.0 | 2.0 |
| Sodium CMC-12 | 0.8 | 0.8 | 0.8 |
| Xanthan Gum | 0.3 | 0.3 | 0.3 |
| Sorbitol | 40.0 | 39.8 | 39.8 |
| Glycerin | 4.0 | 4.0 | 4.0 |
| Tetrasodium pyrophosphate (fine) | 2.0 | 2.0 | 2.0 |
| High Cleaning Silica | 10.0 | 10.0 | 10.0 |
| Macrocrystalline Cellulose/Sodium CMC NF | 1.0 | 1.0 | 1.0 |
| Sodium fluoride | 0.243 | ----- | ----- |
| Stannous Fluoride | ----- | 0.454 | 0.454 |
| Water, sweetener, color, flavoring | QS | QS | QS |
| Abrasive silica | 10.0 | 10.0 | 10.0 |
| Thickener silica | 1.5 | 1.5 | 1.5 |
| Cocamidopropyl Betaine | 1.25 | 1.25 | 1.25 |
| Sodium lauryl sulfate | 1.5 | 1.5 | 1.5 |
| Stannous Phosphate tribasic | 1.0 | ----- | 1.0 |
| Zinc Phosphate | ----- | 1.0 | ----- |

### Example 2

The efficacy of the formulae of Example 1 and Comparative Example A were evaluated as follows:
1. Dental plaque was collected from 4 healthy volunteers and pooled together as inoculum. The optical density ("O.D.") of the inoculum was matched to 0.3 absorbance at 610nm.
2. Sterile vertical hydroxyapatite ("HAP") disks on ACTA lid were incubated under anaerobic conditions at 37 °C for 24 hours with 1 ml of sterile artificial saliva (with 0,01% sucrose) and 1 ml of pooled saliva in a 24 well microplate. The ACTA lid was used to perform simultaneous treatment of all the wells.
3. Freshly prepared treatment solution of 1 part dentifrice of the example being tested to 2 parts sterile distilled water was added to the well and allowed to contact with the HAP disk for 10 mins.
4. The slurry was replaced with 2 ml of sterile Phosphate Buffered Saline ("PBS") and allowed to contact for 1 min.
5. The liquid phase was removed and replaced by 2 ml of sterile artificial saliva.
6. The disks were treated in triplicates for each control and test dentifrice for 8 days.
7. At intervals of 2, 4 and 8 days the discs were collected aseptically and transferred into half strength pre reduced thioglycollate medium.
8. 100µl of each of the dilution 10-4, dilution 10-5 and dilution 10-6 were plated in duplicates for each disk on Neomycin-Vancomycin (NV) Agar and Sheep Blood Agar for Total Gram negative Anaerobes and Total Anaerobic Bacteria.
9. Plates were surface spread using a sterile spreader and incubated anaerobically @37°C for 72 hours before counting the colonies.
10. The pH was monitored for the entire period of the study using the liquid phase.

Plate counts are recorded in log 10 reduction in Table 2 below. Units are in Average Log 10 CFU/ml, where the average was calculated based on the number of samples tested for each example formulation.

**Table 2**

| | **Neomycin Vancomycin Agar** | **Day 8** |
|---|---|---|
| Example 1 | Toothpaste with Stannous Phosphate | 4.2 |
| Comparative Example A | Toothpaste w ith Mica/ Zn Phosphate and Stannous Fluoride Toothpaste | 4.22 |
| Comparative Example B | Toothpaste without metal salt | 4.45 |

Comparative Example A, as shown in Tables 1 and 2, employed a similar base toothpaste formulation as was used in Example 1, but employed Zn Phosphate and Stannous Fluoride instead of Stannous Phosphate. Comparative Example B was in the form of a toothpaste without a metal salt As shown from the results reported in Table 2, the Example 1 formulation with stannous phosphate provided improved anti-microbial activity compared to the Comparative Example A and Comparative Example B formulations.

### Example 3- Stability Data

Stability testing was performed for the formulations of Examples 1 and 2 and Comparative Example A. Three samples of each formulation were provided. Samples for each formulation were held for 3 months at different temperatures, including at room temperature (about 25°C), 40°C and 49°C. The initial fluoride concentration for each sample was measured at the beginning of the study and then was periodically measured after each month of the study. The results showed that the fluoride source for Example 1 and Comparative Example A formulations were sufficiently stable for commercial applications, with the Example 1 formulation being slightly more stable than the Comparative Example A formulation. The formulation of Example 2 was found to be less stable than the formulation of Example 1.

## Claims

1. An oral care composition, comprising:
at least one metal phosphate, wherein the at least one metal phosphate is stannous phosphate in an amount of from 0.5-4.0% by weight, and the stannous phosphate is added to the oral care composition as a preformed salt and wherein the phosphate is not a pyrophosphate, metaphosphate, or other polyphosphate; and
at least one fluoride ion source comprising sodium fluoride, wherein the fluoride ion source is present in an amount of from 0.1-1.0% by weight.

2. The oral care composition of claim 1, wherein the dentifrice composition further comprises an abrasive.

3. The oral care composition of claim 1, further comprising one or more humectants and one or more surfactants, and one or more whitening agents.

4. The oral care composition of claim 1, further comprising an effective amount of one or more alkali phosphate salts that is different from the at least one metal phosphate.

5. The oral care composition of claim 1, with the proviso that the oral care composition is essentially free of acyl sarcosinate or salts thereof, an antioxidant, a phytic acid compound, a copper containing compound, ethylenediaminetetracetates and polyorganosilsesquioxane.

6. The oral care composition of claim 1, wherein the oral care composition is a dentifrice, oral gel, lozenge, mint, or chewing gum.

7. The oral care composition of claim 1, wherein the oral care composition comprises:
stannous phosphate from 0.5 to 4% by weight of the oral care composition;
alkali phosphate salts from 1 to 8% by weight of the oral care composition, wherein the alkali phosphate salts are selected from sodium phosphate dibasic, potassium phosphate dibasic, dicalcium phosphate dihydrate, , and mixtures of any two or more of these;
fluoride from 0.05 to 0.5 % by weight of the oral care composition;
and a silica abrasive dentifrice base.

8. The oral care composition of claim 1, further comprising an effective amount of one or more anti-microbial agents.

9. The oral care composition of claim 1, wherein the pH of the composition is acidic.

10. The oral care composition of claim 1, wherein the pH of the composition is basic.

11. An oral care composition according to any one of the foregoing claims for treating or reducing enamel erosion.

## Patentansprüche

1. Mundpflegezusammensetzung, umfassend:
mindestens ein Metallphosphat, wobei das mindestens eine Metallphosphat Zinnphosphat ist, in einer Menge von 0,5 bis 4,0 Gew.-%, und das Zinnphosphat wird der Mundpflegezusammensetzung als vorgeformtes Salz zugegeben und wobei das Phosphat nicht ein Pyrophosphat, Metaphosphat oder anderes Polyphosphat ist; und
mindestens eine Fluoridionenquelle, umfassend Natriumfluorid, wobei die Fluoridionenquelle in einer Menge von 0,1 bis 1,0 Gew.-% vorliegt.

2. Mundpflegezusammensetzung nach Anspruch 1, wobei die Mundpflegezusammensetzung weiterhin ein Abrasivsstoff umfasst.

3. Mundpflegezusammensetzung nach Anspruch 1, weiterhin umfassend ein oder mehrere Befeuchtungsmittel und ein oder mehrere Tenside und ein oder mehrere Aufhellungsmittel.

4. Mundpflegezusammensetzung nach Anspruch 1, weiterhin umfassend eine wirksame Menge eines oder mehrere Alkaliphosphatsalze, das sich von dem mindestens einen Metallphosphat unterscheidet.

5. Mundpflegezusammensetzung nach Anspruch 1, mit der Maßgabe, dass die Mundpflegezusammensetzung im Wesentlichen frei von Acylsarcosinat oder deren Salze, von einem Antioxidationsmittel, Phytinsäureverbindung, einer kupferenthaltenden Verbindung, Ethylendiamintetracetaten und Polyorganosilsesquioxan ist.

6. Mundpflegezusammensetzung nach Anspruch 1, wobei die Mundpflegezusammensetzung ein Zahnputzmittel, ein orales Gel, eine Pastille, ein Minzbonbon oder Kaugummi ist.

7. Mundpflegezusammensetzung nach Anspruch 1, wobei die Mundpflegezusammensetzung umfasst: Zinnphosphat von 0,5 bis 4 Gew.-% der Mundpflegezusammensetzung;
Alkaliphosphatsalze von 1 bis 8 Gew.-% der Mundpflegezusammensetzung, wobei die Alkaliphosphatsalze ausgewählt sind aus Natriumphosphat dibasisch, Kaliumphosphat dibasisch, Dicalciumphosphatdihydrat und Mischungen von beliebigen zwei oder mehreren von diesen;
Fluorid von 0,05 bis 0,5 Gew.-% der Mundpflegezusammensetzung; und eine Silicabrasivstoffzahnputzmittelbasis.

8. Mundpflegezusammensetzung nach Anspruch 1, weiterhin umfassend eine wirksame Menge von einem oder mehreren antimikrobiellen Mitteln.

9. Mundpflegezusammensetzung nach Anspruch 1, wobei der pH-Wert der Zusammensetzung sauer ist.

10. Mundpflegezusammensetzung nach Anspruch 1, wobei der pH-Wert der Zusammensetzung basisch ist.

11. Mundpflegezusammensetzung nach irgendeinem der vorhergehenden Ansprüche zur Behandlung oder Verminderung von Zahnschmelzerosion.

## Revendications

1. Composition de soin bucco-dentaire, comprenant :
au moins un phosphate métallique, dans laquelle l'au moins un phosphate métallique est du phosphate stanneux en une quantité de 0,5 à 4,0 % en poids, et le phosphate stanneux est ajouté à la composition de soin bucco-dentaire sous forme d'un sel préformé et dans laquelle le phosphate n'est pas un pyrophosphate, un métaphosphate ou un autre polyphosphate ; et
au moins une source d'ions fluorure comprenant le fluorure de sodium, dans laquelle la source d'ions fluorure est présente en une quantité de 0,1 à 1,0 % en poids.

2. Composition de soin bucco-dentaire selon la revendication 1, dans laquelle la composition de dentifrice comprend en outre un abrasif.

3. Composition de soin bucco-dentaire selon la revendication 1, comprenant en outre un ou plusieurs humectants et un ou plusieurs tensioactifs, et un ou plusieurs agents de blanchiment.

4. Composition de soin bucco-dentaire selon la revendication 1, comprenant en outre une quantité efficace d'un ou plusieurs sels de phosphate alcalin qui sont différents de l'au moins un phosphate métallique.

5. Composition de soin bucco-dentaire selon la revendication 1, à la condition que la composition de soin bucco-dentaire soit sensiblement exempte d'acyl sarcosinate ou de sels de ce dernier, d'un antioxydant, d'un composé d'acide phytique, d'un composé contenant du cuivre, d'éthylènediaminetétracétates et de polyorganosilsesquioxane.

6. Composition de soin bucco-dentaire selon la revendication 1, dans laquelle la composition de soin bucco-dentaire est un dentifrice, un gel oral, une pastille, une menthe ou une gomme à mâcher.

7. Composition de soin bucco-dentaire selon la revendication 1, dans laquelle la composition de soin bucco-dentaire comprend :
du phosphate stanneux de 0,5 à 4 % en poids de la composition de soin bucco-dentaire ;
des sels de phosphate alcalin de 1 à 8 % en poids de la composition de soin bucco-dentaire,
dans laquelle les sels de phosphate alcalins sont choisis parmi le phosphate de sodium dibasique, le phosphate de potassium dibasique, le phosphate dicalcique dihydraté et les mélanges de deux ou plus de ceux-ci ;
du fluorure de 0,05 à 0,5 % en poids de la composition de soin bucco-dentaire ;
et une base de dentifrice abrasive à la silice.

8. Composition de soin bucco-dentaire selon la revendication 1, comprenant en outre une quantité efficace d'un ou plusieurs agents antimicrobiens.

9. Composition de soin bucco-dentaire selon la revendication 1, dans laquelle le pH de la composition est acide.

10. Composition de soin bucco-dentaire selon la revendication 1, dans laquelle le pH de la composition est basique.

11. Composition de soin bucco-dentaire selon l'une quelconque des revendications précédentes pour traiter ou réduire l'érosion de l'émail.
